Europäisches Patentamt

European Patent Office

Office européen des brevets

(11) Veröffentlichungsnummer: **0 433 961 A2**

# EUROPÄISCHE PATENTANMELDUNG

(21) Anmeldenummer: 90124525.8

(22) Anmeldetag: **18.12.90**

(51) Int. Cl.5: **A61L 29/00**, A61L 27/00

(30) Priorität: **20.12.89 DE 3942112**

(43) Veröffentlichungstag der Anmeldung:
**26.06.91 Patentblatt 91/26**

(84) Benannte Vertragsstaaten:
**AT BE CH DE DK ES FR GB GR IT LI LU NL SE**

(71) Anmelder: **B. Braun Melsungen AG**
**Carl-Braun Strasse**
**W-3508 Melsungen(DE)**

(72) Erfinder: **Braun, Bernd, Dr. med. Dipl.-Chem.**
**Tränkelücke 1**
**W-3508 Melsungen(DE)**

(74) Vertreter: **von Kreisler, Alek, Dipl.-Chem. et al**
**Patentanwälte Von Kreisler-Selting-Werner,**
**Deichmannhaus am Hauptbahnhof**
**W-5000 Köln 1(DE)**

(54) Medizinische Vorrichtung mit einem oligodynamisch wirkenden Material.

(57) Die Erfindung betrifft eine gewebeverträgliche, medizinische Vorrichtung mit einer oligodynamischen Material, bestehend aus einem Polyurethanwerkstoff und einer bakterizide bzw. mikrobizide Metallionen abgebenden Substanz, die metallisches Silber oder Silbersalze enthält, dadurch gekennzeichnet, daß zur Erhöhung der antimikrobiellen Eigenschaften der Oberfläche und zur gleichzeitigen Erhöhung der radiologischen Nachweisbarkeit in Organen des menschlichen Körpers die oligodynamische Oberfläche neben metallischem Silber zusätzlich Bariumsulfat enthält.

EP 0 433 961 A2

EP 0 433 961 A2

## MEDIZINISCHE VORRICHTUNG MIT EINEM OLIGODYNAMISCH WIRKENDEN MATERIAL

Die vorliegende Erfindung betrifft eine medizinische Vorrichtung aus einem oligodynamischen Material, das antimikrobielle Eigenschaften besitzt. Solche medizinischen Vorrichtungen sind z.B. Verweilkatheter oder Prothesen in Organen des menschlichen Körpers.

Es ist bekannt, daß Katheter und Kanülen aus Kunststoffen bei längerer Liegedauer in Organen des menschlichen Körpers leicht zu einer aszendierenden Infektion des sie umgebenden Gewebes durch Einwanderung von Bakterien führen können. Besonders gravierend sind solche Zwischenfälle im urologischen Bereich, wo Harnröhrenkatheter und Ureterenkatheter routinemäßig verwendet werden.

Weiterhin wird durch die Verwendung dieser aus Kunststoff bestehenden Katheter und Kanülen ein hoher Prozentsatz nosokomialer Infektionen verursacht

Auch im intravasalen Bereich bei der parenteralen Ernährung oder der Katheterisierung zur Patientenüberwachung und medikamentösen Therapie werden unter Verwendung der oben genannten Katheter häufig bakterielle Infektionen beobachtet, die einen dramatischenVerlauf nehmen können. Die Infektionen können z.B. auch eine Thrombosierung im Katheterlumen verursachen, was wiederum Komplikationen für die Patienten zur Folge haben kann.

Man hat versucht, aszendierende Infektionen dadurch auszuschalten, daß medizinische Geräte der genannten Art auf den Oberflächen mit einer Beschichtung bakterizid wirkender Stoffe aus der Gruppe der Desinfektionsmittel versehen wurden. Diese Verwendung bakterizid wirkender chemischer Verbindungen verlief jedoch bei Langzeitanwendungen, z.B. bei chronischen urologischen Erkrankungen, wenig befriedigend, da es zur Ablösung der lediglich mechanisch haftenden Überzüge kommt und keine bakterizide oder bakteriostatische Langzeitwirkung zu erreichen ist. (Lit.Stelle)?

Es ist bekannt, daß Metalle und Metallsalze eine bakterizide oder bakteriostatische Wirkung in physiologischer Elektrolytumgebung aufweisen. Besonders metallisches Silber in verschiedenen Zustandsformen (als Niederschlag, Kolloid, Pulver) und Silbersalze (Silberchlorid, Silbernitrat) ist in diesem Zusammenhang untersucht und angewendet werden.

Die US 4,054,139 beschreibt einen im Kern silberfreien Katheter, auf dessen innerer und äußerer Oberfläche eine freiliegende Beschichtung eines Silber enthaltenden oligodynamischen Materials vorhanden ist. Beispiele für Silber enthaltende oligodynamische Schichten sind Schichten aus metallischem Silber oder organischen Verbindungen wie Silbercitrat und Silberlactat, anorganische Verbindungen wie Silberoxid und Silbernitrat. Als weiteres oligodynamisches Metall wird Gold genannt. Die oligodynamischen Metalle bzw. Metallsalze werden in eine Matrix aus polymerem Material, z.B. Polytetrafluorethylen gemischt. Mit diesem Material wird der Katheter danach beschichtet.

Die EP-0 302 186 offenbart ein gewebeverträgliches, bakterizide, bzw. mikrobizide Metallionen abgebendes medizinisches Gerät mit einer oligodynamischen Oberfläche aus einem Polyurethan-Silicon-Werkstoff, der in der Masse gleichmäßig verteilt die Metallionen abgebende Substanz in Mengen von 1 bis 15 Gew.-% enthält. Als oligodynamisch aktive Substanz wird Silbernitrat eingesetzt.

Die EP 0 301 717 beschreibt Katheter mit antibakterieller und fungizider Wirkung unter Verwendung wasserfreier oder kristallwasserhaltiger Zeolithe, deren Metallionen durch Silber-, Kupfer- oder Zinkionen ersetzt sind. Die so erhaltenen Zeolithe werden in die Kathetermatrix eingelagert. Im Kontakt mit Körperflüssigkeiten werden die oligodynamisch wirksamen Metallionen aus der Kathetermatrix freigesetzt.

EP 0 190 504 verbindet Silber als antibakterielles Mittel mit einem hydratisierten oder hydratisierbaren Oxid als Promoter zur Erhöhung der antibakteriellen Wirkung.

Die im Stand der Technik bekannten Katheter und Prothesen mit oligodynamischer Oberfläche führten zwar zu einer Reduktion der mit ihrer Anwendung in Beziehung stehenden bakteriellen Infektionen, doch ist es eine ständige Forderung der medizinischen Fachwelt, Vorrichtungen mit besseren oligodynamischen Eigenschaften zu entwickeln. Eine weitere Eigenschaft, die diese Vorrichtungen haben sollen, besteht darin, daß sie bei der röntgenologischen Lagekontrolle einen höheren Kontrast zeigen sollen.

Es ist die Aufgabe der vorliegenden Erfindung, Katheter und Prothesen für die medizinische Anwendung bereitzustellen, die mit einer oligodynamischen, bakteriziden Oberfläche versehen sind, und Röntgenkontrast zeigen.

Diese Aufgabe wird gelöst durch eine gewebeverträgliche, medizinische Vorrichtung aus einem oligodynamischen Material, bestehend aus einem Polyurethanwerkstoff und einem bakteriziden bzw. mikrobiziden Metall, dadurch gekennzeichnet, daß zur Erhöhung der antimikrobiellen Eigenschaften und zur gleichzeitigen Erhöhung der radiologischen Nachweisbarkeit in Organen des menschlichen Körpers das oligodynamische Material metallisches Silberpulver und zusätzlich Bariumsulfat enthält.

Es wurde gefunden, daß das oligodynamische Material aus Polyurethanwerkstoff, metallischem Silber-

2

pulver und zusätzlichem Bariumsulfat hervorragende Eigenschaften für den Einsatz in Kathetern oder Prothesen für den medizinischen Gebrauch zeigt.

Die erfindungsgemäßen Vorrichtungen besitzen einerseits gesteigerte röntgenologische Erkennbarkeit. Außerdem wurde andererseits überraschend gefunden, daß die erfindungsgemäßen Vorrichtungen zusätzlich zu dem Vorteil ihrer leichten röntgenologischen Erkennbarkeit hervorragende oligodynamische Oberflächen besitzen, die die antimikrobielle Wirkung von im Stand der Technik bekannten oligodynamischen Oberflächen gegenüber Mikroorganismen weit übertreffen (siehe Beispiel 2). Dabei wirken Silberpulver und Bariumsulfat synergistisch (Beispiel 2).

Es konnte gezeigt werden, daß in der Reihenfolge folgender Katheterwerkstoffe a) thermoplastisches Polyurethan, b) thermoplastisches Polyurethan und Bariumsulfat, c) thermoplastisches Polyurethan plus Silber, d) thermoplastisches Polyurethan plus Bariumsulfat plus metallisches Silber eine Zunahme der mikrobiellen Hemmwirkung feststellbar ist.

Bevorzugt im Sinne dieser Erfindung sind medizinische Vorrichtungen aus einem oligodynamischen Material, das einen Silbergehalt von 1 bis 4,5 Gew.-%, besonders bevorzugt von 3 bis 4 Gew.-%, bezogen auf das Gesamtmaterial, bestehend aus Polyurethan, Silberpulver und Bariumsulfat, besitzt. Der Bariumsulfatanteil des oligodynamischen Materials beträgt bevorzugt 5 bis 45 Gew.-%, bezogen auf das Gesamtmaterial, besonders bevorzugt aus 10 bis 40 Gew.-% und besonders bevorzugt aus 40 Gew.-%.

Es hat sich gezeigt, daß eine bevorzugte Ausführungsform des gewebeverträglichen medizinischen Geräts im Sinne dieser Erfindung oligodynamisches Material aus Polyurethan mit 20 Gew.-% Bariumsulfat und 4 Gew.-% metallischem Silberpulver besitzt.

Hervorragende oligodynamische Eigenschaften und röntgenologische Erkennbarkeit besitzen auch medizinische Vorrichtungen mit einem oligodynamischen Material aus Polyurethan, 40 Gew.-% Bariumsulfat und 2 Gew.-% metallischem Silberpulver. Letztere Zusammensetzung hat gegenüber den im Stand der Technik bekannten Vorrichtungen den Vorteil, daß durch Hinzufügen von Bariumsulfat die Gesamtsilbermenge des oligodynamischen Materials reduziert werden kann, ohne daß die oligodynamische Wirkung abnimmt. Aus den nachfolgenden Ausführungsbeispielen wird hingegen sichtbar, daß diese Vorrichtungen sogar bei beträchtlicher Kostenersparnis durch Reduktion der Silbermenge eine erheblich bessere oligodynamische Wirkung zeigen.

Beispiel 1

100 Gew.-% eines handelsüblichen thermoplastischen Polyurethans, z. B. Pellethane der Amoco Chemicals Co., das sich für medizinische Anwendungen bewährt hat, wird mit 20 Gew.-% bzw. 40 Gew.-% Bariumsulfat (nach Deutschem Arzneibuch, DAB 9) und, wo spezifiziert, mit jeweils 1 Gew.-%, 2 Gew.-% oder 4 Gew.-% Silberpulver mit ca. 5 $\mu$m Korngröße, in einem Mischextruder oder Kunststoffkneter homogen vermischt und die resultierende Schmelze mit den Füllstoffen strangextrudiert und granuliert.

Aus dem Kunststoffgranulat wird nach bekannter Extrusionsverarbeitungsweise ein Schlauch extrudiert, z.B. in den Abmessungen 2 mm Innendurchmesser und 3 mm Außendurchmesser, und aus dem kontinuierlichen Schlauchmaterial der Katheterlänge entsprechende Stücke, z.B. 50 cm lang, geschnitten.

Je nach Verwendungszweck wird an einem oder beiden Katheterschlauchenden angerundet oder geschlossen verrundet und gegebenenfalls mit einem Katheteransatz als Verbindungselement zu Zu- und Ableitungen versehen. (Transurethale Katheter mit Ansatz und Ureterkatheter zur Implantation ohne Katheteransatz).

Beispiel 2

Im in vitro Versuch wird eine Escherichia coli Suspension (ATCC, 11229) mit physiologischer Natriumchloridlösung 1 : 100 auf eine Keimzahl von ca. $2 \times 10^7$ pro Milliliter verdünnt und aus dieser Stammsuspension jeweils 1 ml zu verflüssigtem CASO-Agar gegeben, mit diesen gut vermischt und auf die in sterilen Petrischalen befindlichen Prüfkörper, die aus den zu untersuchenden Werkstoffkomponenten hergestellt werden, gegeben. Die Prüfkörperscheiben (2 mm x 4.5 cm o) waren aus Werkstoffen folgender Zusammensetzung hergestellt:

1 thermoplastisches Polyurethan
(PUR)

2 thermoplastisches Polyurethan, 20 Gew.-% Bariumsulfat
(PUR + 20 BaSO$_4$)

3 thermoplastisches Polyurethan, 20 Gew.-% Bariumsulfat,
1 Gew.-% Silber

3

(PUR + 20 BaSO$_4$ + 1 Ag)

4 thermoplastisches Polyurethan, 20 Gew.-% Bariumsulfat, 2 Gew.-% Silber
(PUR + 20 BaSO$_4$ + 2 Ag)

5 thermoplastisches Polyurethan, 20 Gew.-% Bariumsulfat, 4 Gew.-% Silber
(PUR + 20 BaSO$_4$ + 4 Ag)

6 thermoplastisches Polyurethan, 1 Gew.-% Silber
(PUR + 1 Ag)

7 thermoplastisches Polyurethan, 2 Gew.-% Silber
(PUR + 2 Ag)

8 thermoplastisches Polyurethan, 4 Gew.-% Silber
(PUR + 4 Ag)

9 thermoplastisches Polyurethan, 40 Gew.-% Bariumsulfat, 2 Gew.-% Silber
(PUR + 40 BaSO$_4$ + 2 Ag)

Nach üblicher mikrobiologischer Verfahrensweise wurde aus den bebrüteten Proben die Keimzahl ermittelt.

Aus der Keimzahl konnte in der Reihenfolge der Werkstoffprüfkörper eine Verminderung der Keimzahlen und damit die prozentuale Hemmwirkung (% Reduktion) festgestellt werden (Tab. 1)

## Tab. 1:

| Werkstoff | E.coli (% Wachstumsreduktion) |
|---|---|
| 1 (Kontrolle) * | — |
| 2 PUR + 20 BaSO$_4$ | 8 % |
| 3 PUR + 1 Ag | 7 % |
| 4 PUR + 2 Ag | 11 % |
| 5 PUR + 4 Ag | 15 % |
| 6 PUR + 20 BaSO$_4$ + 1 Ag | 17 % |
| 7 PUR + 20 BaSO$_4$ + 2 Ag | 22 % |
| 8 PUR + 20 BaSO$_4$ + 4 Ag | 26 % |
| 9 PUR + 40 BaSO$_4$ + 2 Ag | 32 % |

(* Werte bezogen auf PUR alleine)

## Ansprüche

1. Gewebeverträgliche, medizinische Vorrichtung aus einem oligodynamisch wirkenden Material, bestehend aus einem Polyurethanwerkstoff und einem bakteriziden bzw. mikrobiziden Metall, dadurch gekennzeichnet, daß zur Erhöhung der antimikrobiellen Eigenschaften der Oberfläche und zur gleichzeitigen Erhöhung der radiologischen Nachweisbarkeit in Organen des menschlichen Körpers das oligodynamisch wirkende Material metallisches Silberpulver und zusätzlich Bariumsulfat enthält.

2. Gewebeverträgliche, medizinische Vorrichtung nach Anspruch 1, dadurch gekennzeichnet, daß der Silbergehalt des oligodynamisch wirkenden Materials 1 bis 4,5 Gew.-% , bezogen auf das Gesamtgewicht, beträgt.

3. Gewebeverträgliche, medizinische Vorrichtung nach Anspruch 1, dadurch gekennzeichnet, daß der Bariumsulfatgehalt des oligodynamisch wirkenden Materials 5 bis 45 Gew.-%, insbesondere 10 bis 40 Gew.-% und besonders bevorzugt 40 Gew.-%, bezogen auf das Gesamtgewicht, beträgt.

4. Gewebeverträgliche, medizinische Vorrichtung nach Anspruch 1, dadurch gekennzeichnet, daß das oligodynamisch wirkende Material aus Polyurethan, 20 Gew.-% Bariumsulfat und 4 Gew.-% metallischem Silber besteht.

5. Gewebeverträgliche, medizinische Vorrichtung nach Anspruch 1, dadurch gekennzeichnet, daß das oligodynamisch wirkende Material aus Polyurethan, 40 Gew.-% Bariumsulfat und 2 Gew.-% metallischem Silber besteht.